# NEUE EUROPÄISCHE PATENTSCHRIFT

(11) **EP 0 559 053 B2**
(45) Veröffentlichungstag und Bekanntmachung des Hinweises auf die Entscheidung über den Einspruch: **14.12.2005**
(45) Hinweis auf die Patenterteilung: 07.05.1997
(21) Anmeldenummer: 93102815.3
(22) Anmeldetag: 24.02.1993
(51) Int. Cl.: C07C 31/125, C07C 211/07, C07C 29/149, C07C 209/48, B01J 23/80, B01J 23/84, B01J 35/10

(54) **Verfahren zur Herstellung von Alkoholen oder Aminen**
Process for the preparation of alcohols or amines
Procédé pour la préparation d'alcools ou d'amines

(30) Priorität: 04.03.1992 DE 4206750
(43) Veröffentlichungstag der Anmeldung: 08.09.1993
(73) Patentinhaber: Johnson Matthey PLC, London SW1Y 5BQ (GB)
(72) Erfinder: Horn, Gerhardt, Dr. Dipl.-Chem., W-4200 Oberhausen (DE); Frohning, Carl Dieter, Dr. Dipl.-Chem., W-4230 Wesel (DE)
(74) Vertreter: Gibson, Sara Hillary Margaret

(56) Entgegenhaltungen:
- EP-A- 0 125 689
- EP-A- 0 175 558
- EP-A- 0 424 069
- EP-A- 0 434 062
- WO-A-82/03854
- US-A- 4 199 479
- US-A- 4 283 581
- C. Pierce. J. Phys. Chem., 57, 1953

## Beschreibung

Die vorliegende Erfindung betrifft ein Verfahren zur Herstellung von Alkoholen oder Aminen durch Umsetzung von Estern, Fettsäuren oder Nitrilen mit Wasserstoff bei erhöhter Temperatur und unter Druck in Gegenwart eines Kupfer enthaltenden Katalysators. Der Katalysator zeichnet sich durch eine vergleichsweise hohe BET-Gesamtoberfläche und eine bestimmte Porenradienverteilung, ausgedrückt durch den Anteil der BET-Gesamtoberfläche, der auf Poren einer bestimmten Größe zurückgeht, aus. Man stellt den Katalysator her, indem man aus einer wäßrigen Lösung, die ein Kupfersalz und Salze weiterer Elemente enthält, mittels eines basischen Fällungsmittels ein Copräzipitat ausfällt, das Copräzipitat anschließend wäscht, trocknet und kalziniert. Der Katalysator enthält kein chemisch gebundenes Chrom.

Kupferhaltige Katalysatoren werden in erheblichem Umfange in technischen Prozessen eingesetzt. Sie spielen beispielsweise bei Hydrierungs- und Dehydrierungsverfahren eine bedeutende Rolle. Hierbei wird der umzusetzende Einsatzstoff dem festangeordneten Katalysator entweder in gasförmigem Zustand (Gasphasefahrweise) oder in flüssigem Zustand (Flüssigphasefahrweise) zugeleitet. Der Katalysator kann auch in feinverteiltem Zustand als Aufschlämmung (Suspensionsfahrweise) genutzt werden.

Eine recht breite Anwendung haben Katalysatoren, die neben Kupfer noch Chrom enthalten, unter anderem für die Hydrierung von Estern gefunden. Diese Katalysatoren sind auch unter der Bezeichnung Kupferchromit- oder Adkins-Katalysatoren bekannt.

Die Verwendung von Adkins-Katalysatoren ist allerdings nicht unproblematisch, da bei ihrer Herstellung Chrom (VI)-Verbindungen, die als krebserregend einzustufen sind und entsprechende Schutzmaßnahmen bei der Handhabung erfordern, eingesetzt werden. Zudem fallen im Verlauf der Herstellung in größeren Mengen Abwässer, die stark mit Kupfer-, Chrom (VI)- und Ammoniumverbindungen belastet sind, an. Derartige Abwässer sind unerwünscht, da sowohl Kupfer- als auch Chrom (VI)-Verbindungen gegenüber Mikroorganismen hochtoxisch wirken und mittels einer aufwendigen Behandlung aus dem Abwasser entfernt werden müssen.

Vereinzelt werden auch Kupferkatalysatoren, die kein chemisch gebundenes Chrom aufweisen, für die Hydrierung von Estern eingesetzt. Nach Lehre der US-A 4 199 479 eignen sich hierfür Kupfer, Zinkoxid und gegebenenfalls Kobalt enthaltende Trägerkatalysatoren. Die EP-B 0 074 193 beschreibt ein Verfahren zur Hydrierung von Estern in der Gasphase mittels eines Kupfer und Zinkoxid enthaltenden Katalysators.

Daneben werden chromfreie Kupferkatalysatoren häufig für andere Umsetzungen empfohlen.

So beschreibt die DE-OS 20 56 612 einen aus Mischkristallen der Reihe (CuₓZn_{y})Al₂(OH)₁₆ CO₃ • 4 H₂O bestehenden Katalysator, wobei x und y Zahlenwerte von 0,5 bis 5,5 annehmen können und die Summe x und y gleich 6 ist. Die Mischkristalle erhält man durch Fällung bei einem pH-Wert von 4,5 bis 5,5, indem man eine wäßrige Kupfer-, Zink- und Aluminiumnitrate enthaltende Lösung mit einem basischen Fällungsmittel, beispielsweise einer wäßrigen Na₂CO₃-Lösung, versetzt.

Der in nichtreduzierter Form CuO, ZnO und Al₂O₃ enthaltende Katalysator wird bei der Umsetzung eines aus Kohlenmonoxid, Kohlendioxid und Wasserstoff bestehenden Gasgemisches zu Methanol eingesetzt.

Die EP 0 125 689 betrifft einen CuO, ZnO und Al₂O₃ enthaltenden Katalysator mit einem Atomverhältnis Cu/Zn zwischen 2,8 und 3,8 (entsprechend 26,9 bis 36,5 Gew.-Teile ZnO je 100 Gew.-Teile CuO) und einem Al₂O₃-Anteil von 8 bis 12 Gew.-%. Al₂O₃ wird als kolloidales Aluminiumhydroxid in die Herstellung eingesetzt, Cu und Zn werden durch Fällung aus Metallsalzlösungen in den Katalysator eingefügt. Der Katalysator wird für die Herstellung von Methanol verwendet.

Aus EP-A-0 434 062 ist ein CuO, Al₂O₃ und ZnO enthaltender Katalysator bekannt, der, bezogen auf 100 Gew.-Teile Metall, Cu, Al und Zn in einem Gewichtsverhältnis von 10 - 80 : 1 - 30 : 10 - 80 enthält. Dieser Katalysator kann zur Hydrierung von Estern in der Gasphase eingesetzt werden.

Die EP-A-0 424 069 beschreibt einen Hydrierkatalysator enthaltend CuO, Al₂O₃ und ZnO, wobei mindestens 80 % des Gesamtporenvolumens in diesem Katalysator von Poren gebildet wird, deren Durchmesser größer als 80 Å ist. Dieser Katalysator erzielt bei der Hydrierung von Estern hinsichtlich Umsatz und Selektivität gute aber nicht herausragende Werte.

Der vorliegenden Erfindung liegt die Aufgabe zugrunde, ein Verfahren bereitzustellen, das nicht nur die Herstellung von Alkoholen durch katalytische Hydrierung von Estern und Fettsäuren, sondern auch die Herstellung von Aminen durch katalytische Hydrierung von Nitrilen mittels eines chromfreien Kupferkatalysators ermöglicht. Das Verfahren soll darüber hinaus sicherstellen, daß die Reaktion, insbesondere bei höheren Temperaturen, mit hohem Umsatz und hoher Selektivität abläuft und die Bildung von Nebenprodukten weitestgehend vermieden wird, wobei die mit üblichen Adkins-Katalysatoren erzielbaren Ergebnisse nicht nur erreicht, sondern in vielen Fällen sogar deutlich verbessert werden. Ein weiterer Vorteil besteht darin, daß bei Herstellung des chromfreien Kupferkatalysators Probleme, die bei der Herstellung von Adkins-Katalysatoren eine Rolle spielen, wie die Handhabung krebserregender Chrom (VI)-Verbindungen, Anfall von schädliche Verbindungen enthaltenden Abwässern und Entsorgung chromhaltiger Altkatalysatoren, umgangen werden.

Die Aufgaben werden gelöst durch ein Verfahren zur Herstellung von Alkoholen oder Aminen durch Umsetzung von Estern, Fettsäuren oder Nitrilen mit Wasserstoff unter Druck und erhöhter Temperatur in Gegenwart eines Kupfer enthaltenden Katalysators, dadurch gekennzeichnet, daß der Katalysator im nichtreduzierten Zustand je 100 Gew.-Teile CuO 40 bis 130 Gew.-Teile ZnO, 2 bis 50 Gew.-Teile Al₂O₃ und gegebenenfalls 0,5 bis 8 Gew.-Teile Mn-, Mo-, V-, Zr- und/oder Erdalkalimetall-Oxid enthält und eine BET-Gesamtoberfläche von 80 bis 175 m²/g Katalysator im nichtreduzierten Zustand aufweist, wobei 75 bis 95 % der BET-Gesamtoberfläche von Poren eines Radius rₚ ≤ 15 nm gebildet werden.

Ein weiteres Merkmal des erfindungsgemäßen Verfahrens ist eine relativ große aktive Kupfermetalloberfläche des Katalysators. Sie beträgt im reduzierten Katalysator 30 bis 125, insbesondere 35 bis 100, bevorzugt 40 bis 85 m²/g Cu und übertrifft damit die aktive Kupfermetalloberfläche entsprechender Kupferchromit-Katalysatoren. Die Bestimmungsmethode ist M.J. Juys, P.H. van Oeffelt, W.G.J. Brouwe, A.P. Pijpers und J.J.F. Scholten, Applied Catalysis, 46 (1989), Seiten 161 bis 173 zu entnehmen.

Der Katalysator enthält je 100 Gew.-Teile CuO 40 bis 130, insbesondere 45 bis 100, bevorzugt 45 bis 80 Gew.-Teile ZnO und 2 bis 50, insbesondere 3 bis 40, bevorzugt 4 bis 30, besonders bevorzugt 4 bis 11 Gew.-Teile Al₂O₃.

Der Katalysator umfaßt erforderlichenfalls noch weitere Stoffe. Hierunter fallen Oxide des Mangans, Molybdäns, Vanadiums, Zirkons und/oder eines Erdalkalimetalls. Je 100 Gew.-Teile CuO beträgt ihr Anteil 0,5 bis 8, insbesondere 1 bis 6, bevorzugt 2 bis 4 Gew.-Teile gerechnet als MnO, MoO₃, V₂O₅, ZrO₂ und MeO, worin Me für ein Erdalkalimetall steht.

Besonders geeignet als weitere Stoffe sind Mangan- und/oder ein Erdalkalimetall-Oxid. Als Erdalkalimetall-Oxid kommen Mg-, Ca- oder Ba-Oxid, insbesondere Ca- oder Ba-Oxid, bevorzugt Ba-Oxid in Betracht.

Ein weiteres Merkmal des erfindungsgemäßen Verfahrens ist die vergleichsweise hohe BET-Gesamtoberfläche des Katalysators. Sie beträgt 80 bis 175, insbesondere 85 bis 160, bevorzugt 90 bis 155 m²/g Katalysator im nichtreduzierten Zustand.

Unter BET-Gesamtoberfläche wird die durch Adsorption von Stickstoff nach der Brunauer, Emmett und Teller-Methode (BET) ermittelte Oberfläche verstanden. Das Verfahren zur Bestimmung der BET-Gesamtoberfläche ist in J. Amer. Chem. Soc., 60, (1938) 309 beschrieben.

Ein zusätzliches Merkmal des erfindungsgemäß eingesetzten Katalysators ist eine bestimmte Verteilung der Porenradien, ausgedrückt durch einen hohen Anteil der BET-Gesamtoberfläche, der von Poren eines Radius rₚ ≤ 15 nm (150 Å) gebildet wird. Ihr Anteil beträgt 75 bis 95, insbesondere 80 bis 92, bevorzugt 84 bis 90 % der BET-Gesamtoberfläche.

50 bis 85, insbesondere 60 bis 80 % der BET-Gesamtoberfläche werden von Poren eines Radius rₚ ≤ 9 nm (90 Å) gebildet.

Auf den Bereich der Poren, deren Radius rₚ = 9 bis 15 nm beträgt, entfallen 5 bis 45, insbesondere 15 bis 40, bevorzugt 18 bis 30 % der BET-Gesamtoberfläche.

An dieser Stelle sei nochmals darauf hingewiesen, daß die vorangegangenen Angaben zur BET-Gesamtoberfläche sich jeweils auf den Katalysator in nichtreduzierter Form beziehen.

Die Bestimmung der Porenradien erfolgt durch Auswertung der Desorptionsisothermen mit Hilfe der Kelvin-Gleichung gemäß C. Pierce, J. Phys. Chem. 57, (1953) 149. Die Angaben hinsichtlich der Porenradien gehen ebenfalls auf den nichtreduzierten Katalysator zurück.

Der Katalysator kann, falls gewünscht, neben den bereits erwähnten Bestandteilen noch einen Träger aufweisen. Je 100 Gew.-Teile CuO enthält er 2 bis 80, insbesondere 4 bis 60, bevorzugt 5 bis 35 Gew.-Teile Träger. Als Träger können übliche in Wasser unlösliche Materialien verwendet werden. Geeignete Trägermaterialien sind SiO₂, Kieselgur, Kieselgel und Al₂O₃, insbesondere Al₂O₃.

Der Katalysator enthält in reduzierter Form je 100 Gew.-Teile Cu 48 bis 163, insbesondere 56 bis 125, bevorzugt 56 bis 100 Gew.-Teile ZnO, 2,4 bis 63, insbesondere 3,7 bis 50, bevorzugt 5,0 bis 37,5, besonders bevorzugt 5 bis 13,8 Gew.Teile Al₂O₃ und gegebenenfalls 0,6 bis 10, insbesondere 1,2 bis 7,5, bevorzugt 2,4 bis 5,0 Gew.-Teile Mn-, Mo-, V-, Zr- und/oder Erdalkalimetalloxid.

Im folgenden soll auch die Herstellung des chromfreien Kupferkatalysators näher beschrieben werden. Man geht von einer wäßrigen Kupfer-, Zink-, Aluminium- und gegebenenfalls Mn-, Mo-, V-, Zr- und/oder Erdalkalimetallsalze enthaltenden, wäßrigen Lösung aus. Diese, im folgenden auch als Mischsalzlösung bezeichnete Lösung enthält 10 bis 100 g Cu/l, 10 bis 50 g Zn/l und Al entsprechend 2 bis 80 g Al₂O₃/l. Falls erforderlich weist die Mischsalzlösung je Liter noch 3 bis 80 g Mn, Mo, V, Zr und/oder Erdalkalimetall, gerechnet als MnO, MoO₃, V₂O₅, ZrO₂ und MeO, worin Me für ein Erdalkalimetall steht, auf.

Die Mischsalzlösung stellt man durch Lösen wasserlöslicher Salze der vorstehend genannten Elemente in Wasser her. Besonders bewährt hat es sich, Nitrate zu verwenden.

Es empfiehlt sich, die Mischsalzlösung, gegebenenfalls durch Zugabe von Säure, auf einen pH-Wert unterhalb 4,0 einzustellen.

Als Fällungsmittel dient eine wäßrige Lösung einer basischen Verbindung, üblicherweise einewäßrige Alkalicarbonat- oder Alkalihydrogencarbonatlösung.

Um eine möglichst vollständige Fällung sicherzustellen und zugleich ein besonders homogenes Copräzipitat zu erhalten, setzt man die basische Verbindung im stöchiometrischen Überschuß ein.

Die Fällung wird herbeigeführt, indem man die Mischsalzlösung und das Fällungsmittel voneinander getrennt, aber gleichzeitig, kontinuierlich oder diskontinuierlich unter intensiver Vermischung zusammenführt.

Das Copräzipitat wird durch vergleichsweise langsames Zusammenführen der Mischsalzlösung und des Fällungsmittels gefällt. Die Fällungszeit sollte wenigstens 10 Minuten betragen.

Man arbeitet während der Fällung bei einem konstanten pH-Wert innerhalb eines pH-Bereiches von 6,5 bis 8,5, insbesondere 7,6 bis 8,0. Schwankungen des pH-Wertes sollten möglichst gering gehalten werden.

Die Fällung wird bei konstanten Temperaturen oberhalb 70°C, insbesondere in einem Bereich von 75 bis 95°C durchgeführt.

Im Anschluß an die Fällung wird das Copräzipitat von der Mutterlauge abgetrennt und sorgfältig gewaschen.

Im allgemeinen genügt es, während des Waschens eine Temperatur von 55 bis 85, insbesondere 60 bis 75°C einzuhalten und je kg Copräzipitat 5 bis 50 kg Waschwasser einzusetzen.

Die Dauer des Waschvorganges muß ausreichend bemessen werden. Sie sollte mindestens 60 Minuten betragen.

Es erweist sich als ausreichend, das Copräzipitat bei Temperaturen von 50 bis 120°C zu trocknen, bis ein Restfeuchtegehalt von etwa 2 bis 15 Gew.-% Wasser, bezogen auf das getrocknete Copräzipitat, erreicht wird.

Die anschließende Kalzinierung erfolgt bei 250 bis 450°C über einen Zeitraum von 3 bis 10 Stunden. Der kalzinierte Katalysator kann entweder in Pulverform direkt für Suspensionshydrierungen oder nach Formgebung, wie Tablettierung oder Pelletierung, als festangeordneter Katalysator eingesetzt werden.

Das erfindungsgemäße Verfahren eignet sich einerseits zur Herstellung von Alkoholen durch Hydrierung der entsprechenden Ester oder Fettsäuren und andererseits zur Herstellung von Aminen durch Hydrierung von Nitrilen. Die Einsatzstoffe müssen keine besonderen Bedingungen, beispielsweise hinsichtlich Zusammensetzung oder Reinheit, erfüllen. Sie können in der Qualität, wie sie üblicherweise technisch verfügbar sind, verwendet werden. Sogar Einsatzstoffe, die aufgrund von bestimmten Verunreinigungen, beispielsweise schwefelhaltiger Verbindungen, als schwer hydrierbar gelten, können mit gutem Erfolg in das erfindungsgemäße Verfahren eingesetzt werden.

Das erfindungsgemäße Verfahren erfordert für die Herstellung von Alkoholen etwas andere Reaktionsbedingungen als für die Herstellung von Aminen. Will man Alkohole erzeugen, so setzt man die entsprechenden Ester oder Fettsäuren bei 200 bis 350, insbesondere 220 bis 330, bevorzugt 230 bis 320°C um. Der Druck beträgt üblicherweise 15 bis 40, insbesondere 18 bis 35, bevorzugt 20 bis 32 MPa.

Als Einsatzstoffe dienen Ester von Carbonsäuren mit 2 bis 30 Kohlenstoffatomen, insbesondere von nativen Carbonsäuren, mit 8 bis 30, insbesondere 10 bis 24, bevorzugt 12 bis 22 Kohlenstoffatomen. Hierunter fallen Ester, die von den vorstehend genannten Carbonsäuren und einwertigen Alkoholen mit 1 bis 4 Kohlenstoffatomen einerseits und mehrwertigen Alkoholen mit 2 bis 6 Kohlenstoffatomen andererseits gebildet werden.

Von technischer Bedeutung sind die Methyl- und Butylester der vorstehend genannten Carbonsäuren.

Beispiele für geeignete Ester sind Ölsäuremethylester, Estergemische der Talgfettsaüre, Palmfettsäure, Palmkernfettsäure und Kokosnußfettsäure, insbesondere deren Methylester.

Als Einsatzstoffe lassen sich auch Fettsäuren verwenden. Die Fettsäuren enthalten 8 bis 30, insbesondere 10 bis 24, bevorzugt 12 bis 22 Kohlenstoffatome. Sie können gesättigt, einfach ungesättigt oder mehrfach ungesättigt sein.

Beispiele für geeignete Fettsäuren sind Ölsäure, Talgfettsäure, Palmfettsäure, Palmkernfettsäure und Kokosnußfettsäure.

Zur Herstellung von Aminen setzt man Nitrile üblicherweise unter etwas milderen Bedingungen als die Ester und Fettsäuren um. Temperaturen von 160 bis 250, insbesondere 190 bis 240, bevorzugt 210 bis 230 und Drücke von 0,5 bis 10, insbesondere 1,0 bis 5,0, bevorzugt 1,2 bis 3,0 MPa stellen geeignete Reaktionsbedingungen dar. Es lassen sich Nitrile mit 4 bis 30, insbesondere 8 bis 24, bevorzugt 10 bis 22 Kohlenstoffatomen auf diese Weise in die entsprechenden Amine überführen.

Beispiele für geeignete Nitrile sind Propionitril, Butyronitril, Valeronitril, Capronsäurenitril, Caprilsäurenitril, Laurinsäurenitril, Myristinsäurenitril, Palminsäurenitril, Ölsäurenitril, Stearinsäurenitril, Alkylaminonitrile mit 2 bis 6 Kohlenstoffatomen in der Alkylkette und aliphatische, cycloaliphatische und aromatische Dinitrile, wie Adipinsäuredinitril und Isophorondinitril.

### Experimenteller Teil

### Beispiel 1

### Hydrierung eines überwiegend Ölsäuremethylester enthaltenden Fetbsäuremethylester-Gemisches

Als Einsatzstoff dient ein überwiegend aus Ölsäuremethylester bestehendes Fettsäuremethylester-Gemisch (Bezeichnung Estol 1400, Handelsprodukt der Firma Unilever), das folgende Zusammensetzung (ermittelt durch gaschromatografische Analyse) aufweist:

| | |
|---|---|
| C₁₂ - Säure-methylester | 0,5 Gew.-% |
| C₁₄ - Säure-methylester | 4,5 Gew.-% |
| C₁₆ - Säure-methylester | 11,5 Gew.-% |
| C₁₈ - Säure-methylester ca. | 81,0 Gew.-% |
| C₂₀ - Säure-methylester ca. | 0,8 Gew.-% |
| (Restliche Komponenten nicht identifiziert) | |

Das Fettsäuremethylester-Gemisch ist aufgrund seines Schwefelgehaltes (25 Gew.-ppm S) schwierig zu hydrieren.

Den für die Umsetzung benötigten Katalysator I stellt man folgendermaßen her:

Durch Auflösen entsprechender Mengen Cu(NO₃)₂ • 3H₂O, Zn(NO₃)₂ • 6H₂O und Al(NO₃)₃ • 9H₂O in Wasser bereitet man eine Lösung (Mischsalzlösung). Als basisches Fällungsmittel dient eine wäßrige Na₂CO₃-Lösung. Man führt die auf 80°C erhitzte Mischsalzlösung und das ebenfalls auf 80°C erhitzte basische Fällungsmittel getrennt, aber gleichzeitig unter Rühren zusammen, filtriert und wäscht den Niederschlag (Copräzipitat) mit heißem Wasser. Der gewaschene Filterkuchen wird auf eine Endfeuchte von ≤ 5 Gew.-%, bezogen auf Katalysatormasse, getrocknet und anschließend im N₂-Strom bei 380°C kalziniert.

Der für die Umsetzung verwendete, kalzinierte Katalysator I weist in nichtreduziertem Zustand 59,2 Gew.-% CuO (entsprechend 47,3 Gew.-% Cu) auf und enthält je 100 Gew.-Teile CuO 52,4 Gew.-Teile ZnO und 8,4 Gew.-Teile Al₂O₃.

Die BET-Gesamtoberfläche beträgt 126 m²/g Katalysator in nichtreduziertem Zustand. 89 % der BET-Gesamtoberfläche werden von Poren eines Radius rₚ ≤ 15 nm und 76 % der BET-Gesamtoberfläche werden von Poren eines Radius rₚ ≤ 9 nm gebildet. Die Kupfermetalloberfläche des reduzierten Katalysators liegt bei 78 m²/g Cu.

In einem mit einem Magnethubrührer bestückten Autoklaven (Volumen 1 Liter) werden unter Luftausschluß 400 g des vorstehend beschriebenen Fettsäuremethylester-Gemisches und 4 g Katalysator I vorgeleat. Anschließend wird Wasserstoff bis zu einem Druck von 18 MPa aufgepreßt und unter Rühren (100 Hübe/Minute) binnen 50 Minuten auf 250°C aufgeheizt. Der Druck steigt während des Aufheizens auf 25 MPa. Durch wiederholtes Aufpressen von Wasserstoff hält man den Druck auf 25 MPa konstant. Nach 125 Minuten Reaktionszeit wird kein Wasserstoff mehr aufgenommen. Die Reaktion ist beendet. Der Autoklav wird entspannt und abgekühlt.

### Vergleichsbeispiele 1 bis 5

### Hydrierung eines überwiegend Ölsäuremethylester enthaltenden Fettsäuremethylester-Gemisches

Es wird, wie in Beispiel 1 angegeben, gearbeitet, jedoch werden jeweils 4 g eines handelsüblichen Katalysators eingesetzt. Die Umsetzung wird nach 125 Minuten abgebrochen. Die Wasserstoffaufnahme ist zu diesem Zeitpunkt noch nicht beendet.

Folgende Katalysatoren werden eingesetzt:
Katalysator A in Vergleichsbeispiel 1:
   Ein Kupferchromit-Katalysator, der 42 Gew.-% Cu und 26 Gew.-% Cr enthält.
Katalysator B in Vergleichsbeispiel 2:
   Ein Kupferchromit-Katalysator, der 36 Gew.-% Cu, 32 Gew.-% Cr, 2,2 Gew.-% Ba und 2,4 Gew.-% Mn enthält. Die BET-Gesamtoberfläche beträgt 30 m²/g Katalysator.
Katalysator C in Vergleichsbeispiel 3:
   Ein Kupferchromit-Katalysator, der 36 Gew.-% Cu, 32 Gew.-% Cr, 2,2 Gew.-% Ba und 2,4 Gew.-% Mn enthält. Die BET-Gesamtoberfläche beträgt 65 m²/g Katalysator.
Katalysator D in Vergleichsbeispiel 4:
   Ein Kupferchromit-Katalysator, der 36 Gew.-% Cu, 33 Gew.-% Cr und 3 Gew.-% Mn enthält.
Katalysator E in Vergleichsbeispiel 5:
   Ein Kupferchromit-Katalysator, der etwa 47 Gew.-% CuO, etwa 49 Gew.-% Cr₂O₃ und etwa 4 Gew.-% MnO₂ enthaft. Die BET-Gesamtoberfläche beträgt 30 m²/g Katalysator.

### Beispiele 2

### Hydrierung eines überwiegend Ölsäuremethylester enthaltenden Fettsäuremethylester-Gemisches

Man arbeitet wie in Beispiel 1 angegeben, führt jedoch die Reaktion bei 300°C und 27 MPa durch. Die Hydrierung ist bereits nach 45 Minuten beendet.

### Vergleichsbeispiel 6

### Hydrierung eines überwiegend Ölsäuremethylester enthaltenden Fettsäuremethylester-Gemisches

Es wird, wie in Beispiel 2 angegeben, gearbeitet, jedoch werden 4 g Katalysator B eingesetzt. Die Wasserstoffaufnahme ist allerdings erst nach 90 Minuten beendet.

Die Ergebnisse der Beispiele 1 und 2 sowie der Vergleichsbeispiele 1 bis 6 sind in der nachfolgenden Tabelle 1 zusammengestellt.

**Tabelle 1**

| | Beispiel 1 | Vergleichsbeispiele | | | | | Beispiel 2 | Vergleichsbeispiel 6 |
|---|---|---|---|---|---|---|---|---|
| | | 1 | 2 | 3 | 4 | 5 | | |
| Katalysator | I | A | B | C | D | E | I | B |
| Temperatur (°C) | 250 | 250 | 250 | 250 | 250 | 250 | 300 | 300 |
| Druck (MPa) | 25 | 25 | 25 | 25 | 25 | 25 | 27 | 27 |
| Reaktionszeit (min) | 125 | 125 | 125 | 125 | 125 | 125 | 45 | 90 |

| Zusammensetzung des Reaktionsproduktes (in Gew.-%) | | | | | | | | |
|---|---|---|---|---|---|---|---|---|
| C₁₂ - Alkohol | 0,5 | 0,3 | 0,4 | 0,4 | 0,3 | 0,3 | 0,5 | 0,5 |
| C₁₄ - Alkohol | 4,3 | 2,9 | 1,4 | 1,3 | 1,8 | 1,0 | 4,5 | 3,5 |
| C₁₆ - Säure-methylester | 0,2 | 10,5 | 9,1 | 9,3 | 7,1 | 8,7 | 0,1 | 1,3 |
| C₁₆ - Alkohol | 11,2 | 0,7 | 2,0 | 2,6 | 3,9 | 1,9 | 10,8 | 10,1 |
| C₁₈ - Säure-methylester | 1,3 | 74,9 | 66,0 | 59,6 | 57,2 | 67,6 | 1,0 | 2,8 |
| C₁₈ - Alkohol | 78,2 | 3,7 | 13,0 | 6,5 | 22,6 | 11,4 | 78,7 | 75,5 |
| C₂₀ - Alkohol | 0,6 | 0,2 | 0,3 | 0,2 | 0,1 | 0,3 | 0,5 | 0,3 |

### Beispiel 3

### Hydrierung eines überwiegend Laurin-und Myristinsäuremethylester enthaltenden Fettsäuremethylester-Gemisches

Als Einsatzstoff dient ein überwiegend aus Laurinsäuremethylester (C₁₂-Säure-methylester) und aus Myristinsäuremethylester (C₁₄-Säure-methylester) bestehendes Fettsäuremethylester-Gemisch (Cocos-Methylester), das folgende Zusammensetzung (ermittelt durch gaschromatografische Analyse) aufweist:

| | |
|---|---|
| C₁₀/C₁₁ - Säure-methylester | 0,7 Gew.-% |
| C₁₂ - Säure-methylester ca. | 55,0 Gew.-% |
| C₁₄ - Säure-methylester ca. | 21,0 Gew.-% |
| C₁₆ - Säure-methylester ca. | 10,0 Gew.-% |
| C₁₈ - Säure-methylester ca. | 13,0 Gew.-% |

Den für die Umsetzung benötigten Katalysator II stellt man analog zu der in Beispiel 1 beschriebenen Herstellung des Katalysators I her.

Der kalzinierte Katalysator II weist in nichtreduziertem Zustand 58,6 Gew.-% CuO (entsprechend 46,9 Gew.-% Cu) auf und enthält je 100 Gew.-Teile CuO 50,2 Gew.-Teile Zno, 8,0 Gew.-Teile Al₂O₃ und 2,6 Gew.-Teile BaO.

Die BET-Gesamtoberfläche beträgt 118 m²/g Katalysator in nichtreduziertem Zustand. 87 % der BET-Gesamtoberfläche werden von Poren eines Radius rₚ ≤ 15 nm und 74 % der BET-Gesamtoberfläche werden von Poren eines Radius rₚ ≤ 9 nm gebildet. Die Kupfermetalloberfläche des reduzierten Katalysators liegt bei 74 m²/g Cu.

In einem mit einem Magnethubrührer bestückten Autoklaven (Volumen 1 Liter) werden unter Luftausschluß 400 g des vorstehend beschriebenen Fettsäuremethylester-Gemisches (Cocos-Methylester) und 4 g Katalysator II vorgelegt. Anschließend wird Wasserstoff bis zu einem Druck von 18 MPa aufgepreßt und unter Rühren (100 Hübe/Minute) binnen 50 Minuten auf 250°C aufgeheizt. Der Druck steigt während des Aufheizens auf 25 MPa. Durch wiederholtes Aufpressen von Wasserstoff hält man den Druck auf 25 MPa konstant. Nach 75 Minuten Reaktionszeit wird kein Wasserstoff mehr aufgenommen. Die Reaktions ist beendet. Der Autoklav wird entspannt und abgekühlt.

### Vergleichsbeispiele 7 und 8

### Hydrierung eines überwiegend Laurin-und Myristinsäuremethylester enthaltenden Fettsäuremethylester-Gemisches

Es wird, wie in Beispiel 3 angegeben, gearbeitet, jedoch werden 4 g Katalysator B (Vergleichsbeispiel 7) und 4 g Katalysator D (Vergleichsbeispiel 8) eingesetzt. In beiden Vergleichsversuchen 7 und 8 wird allerdings erst nach 135 Minuten Reaktionszeit kein Wasserstoff aufgenommen.

Die Ergebnisse des Beispiels 3 und der Vergleichsbeispiele 7 und 8 sind in der nachfolgenden Tabelle 2 zusammengestellt.

**Tabelle 2**

| | Beispiel 3 | Vergleichsbeispiel | |
|---|---|---|---|
| | | 7 | 8 |
| Katalysator | II | B | D |
| Temperatur (°C) | 250 | 250 | 250 |
| Druck (MPa) | 25 | 25 | 25 |
| Reaktionszeit (min) | 75 | 135 | 135 |

| Zusammensetzung des Reaktionsproduktes (in Gew.-%) | | | |
|---|---|---|---|
| C₁₀₋₁₁- Alkohol | 0,7 | 0,4 | 0,5 |
| C₁₂ - Säure-methylester | 1,0 | 35,3 | 28,6 |
| C₁₂ - Alkohol | ∼54 | 20,7 | 25,5 |
| C₁₄ - Säure-methylester | 0,5 | 14,5 | 12,4 |
| C₁₄ - Alkohol | 20,5 | 6,5 | 8,5 |
| C₁₆ - Säure-methylester | 0,3 | 7,2 | 6,8 |
| C₁₆ - Alkohol | ∼10 | 2,8 | 3,7 |
| C₁₈ - Säure-methylester | 0,4 | 9,4 | 9,7 |
| C₁₈ - Alkohol | 12,5 | 2,9 | 4,1 |

### Beispiel 4

### Hydrierung von Palmkernfettsäure

Als Einsatzstoff dient unveresterte Palmkernfettsäure, die folgende Kennzahlen aufweist:

| | |
|---|---|
| Jod-Zahl | 13,0 g J₂/100 g |
| Säurezahl | 267 mg KOH/g |

In einem mit einem Magnethubrührer bestückten Autoklaven (Volumen 1 Liter) werden unter Luftausschluß 400 g vorstehend beschriebener Palmkernfettsäure und 32 g Katalysator I vorgelegt. Anschließend wird Wasserstoff bis zu einem Druck von 18 MPa aufgepreßt und unter Rühren (100 Hübe/Minute) binnen 90 Minuten auf 300°C aufgeheizt. Der Druck steigt während des Aufheizens auf 27 MPa. Durch wiederholtes Aufpressen von Wasserstoff hält man den Druck auf 27 MPa konstant. Nach 100 Minuten Reaktionszeit wird kein Wasserstoff mehr aufgenommen. Die Reaktion ist beendet. Der Autoklav wird entspannt und abgekühlt.

### Vergleichsbeispiel 9

### Hydrierung vonPalmkernfettsäure

Es wird, wie in Beispiel 4 angegeben, gearbeitet, jedoch werden 32 g eines handelsüblichen Kupferchromit-Katalysators, der 35,5 Gew.-% Cu, 32,6 Gew.-% Cr, 4,2 Gew.-% Ba enthält, (Katalysator F) eingesetzt.

Die Ergebnisse von Beispiel 4 und Vergleichsbeispiel 9 sind in der nachfolgenden Tabelle 3 zusammengestellt.

**Tabelle 3**

| | Beispiel 4 | Vergleichsbeispiel 9 |
|---|---|---|
| Katalysator | I | F |
| Temperatur (°C) | 300 | 300 |
| Druck (MPa) | 27 | 27 |
| Reaktionszeit (min) | 100 | 100 |

| Kennzahlen des Reaktionsproduktes | | |
|---|---|---|
| Jod-Zahl (g J₂/100 g) | 0,12 | 1,9 |
| Säurezahl (mg KOH/g) | 0,39 | 5,8 |
| Hydroxylzahl (mg KOH/g) | 273 | 209 |

### Beispiel 5

### Hydrierung eines Fettsäurenitrils

Als Einsatzstoff dient Talgfettsäurenitril, das eine Jodzahl von 55,3 g J₂/100 g aufweist.

In einem mit einem Magnethubrührer bestückten Autoklaven (Volumen 1 Liter) werden unter Luftausschluß 400 g Talgfettsäurenitril und 10 g Katalysator I vorgelegt. Man führt die Umsetzung bei 220°C und 1,5 MPa durch. Nach 240 Minuten Reaktionszeit wird kein Wasserstoff mehr aufgenommen. Die Reaktion ist beendet. Der Autoklav wird entspannt und abgekühlt.

Das Reaktionsprodukt enthält 63,8 Gew.-% primäres Fettamin, 34,5 Gew.-% sekundäres Fettamin und 1,5 Gew.-% tertiäres Fettamin. Die Rest-Jodzahl beträgt 52,6 g J₂/100 g.

## Patentansprüche

1. Verfahren zur Herstellung von Alkoholen oder Aminen durch Umsetzung von Estern, Fettsäuren oder Nitrilen mit Wasserstoff unter Druck und erhöhter Temperatur in Gegenwart eines Katalysators, welcher im nichtreduzierten Zustand aus je 100 Gew.-Teilen CuO 40 bis 130 Gew.-Teilen ZnO, 2 bis 50 Gew.-Teilen Al₂O₃ und gegebenenfalls 0,5 bis 8 Gew.-Teilen Mn-, Mo-, V-, Zr- und/oder Erdalkalimetall-Oxid besteht, und eine BET-Gesamtoberfläche von 80 bis 175 m²/g Katalysator im nichtreduzierten Zustand aufweist, wobei 75 bis 95 % der BET-Gesamtoberfläche von Poren eines Radius rₚ ≤ 15 nm gebildet werden.

2. Verfahren nach Anspruch 1, **dadurch gekennzeichnet, daß** die aktive Kupfermetalloberfläche des reduzierten Katalysators 30 bis 125, insbesondere 35 bis 100, bevorzugt 40 bis 85 m²/g Cu beträgt.

3. Verfahren nach Anspruch 1 oder 2, **dadurch gekennzeichnet, daß** der Katalysator im nichtreduzierten Zustand aus je 100 Gew.-Teilen CuO 45 bis 100, insbesondere 45 bis 80 Gew.-Teilen ZnO und 3 bis 40, insbesondere 4 bis 30, bevorzugt 4 bis 11 Gew.-Teilen Al₂O₃ besteht.

4. Verfahren nach einem oder mehreren der Ansprüche 1 bis 3, **dadurch gekennzeichnet, daß** der Katalysator eine BET-Gesamtoberfläche von 85 bis 160, insbesondere 90 bis 155 m²/g Katalysator im nichtreduzierten Zustand aufweist.

5. Verfahren nach einem oder mehreren der Ansprüche 1 bis 4, **dadurch gekennzeichnet, daß** 80 bis 92, insbesondere 84 bis 90 % der BET-Gesamtoberfläche von Poren eines Radius rₚ ≤ 15 nm gebildet werden.

6. Verfahren nach einem oder mehreren der Ansprüche 1 bis 5, **dadurch gekennzeichnet, daß** Ester oder Fettsäuren bei 200 bis 350, insbesondere 220 bis 330, bevorzugt 230 bis 320°C und 15 bis 40, insbesondere 18 bis 35, bevorzugt 20 bis 32 MPa umgesetzt werden.

7. Verfahren nach einem oder mehreren der Ansprüche 1 bis 6, **dadurch gekennzeichnet, daß** Ester von Carbonsäuren, insbesondere von nativen Carbonsäuren, mit 8 bis 30, insbesondere 10 bis 24, bevorzugt 12 bis 22 Kohlenstoffatomen umgesetzt werden.

8. Verfahren nach einem oder mehreren der Ansprüche 1 bis 6, **dadurch gekennzeichnet, daß** Fettsäuren mit 8 bis 30, insbesondere 10 bis 24, bevorzugt 12 bis 22 Kohlenstoffatomen umgesetzt werden.

9. Verfahren nach einem oder mehreren der Ansprüche 1 bis 5, **dadurch gekennzeichnet, daß** Nitrile bei 160 bis 250, insbesondere 190 bis 240, bevorzugt 210 bis 230°C und 0,5 bis 10, insbesondere 1,0 bis 5,0, bevorzugt 1,2 bis 3,0 MPa umgesetzt werden.

10. Verfahren nach einem oder mehreren der Ansprüche 1 bis 5 und 9, **dadurch gekennzeichnet, daß** Nitrile mit 4 bis 30, insbesondere 8 bis 24, bevorzugt 10 bis 22 Kohlenstoffatomen umgesetzt werden.

## Claims

1. A process for the preparation of alcohols or amines through the reaction of esters, fatty acids or nitriles with hydrogen under pressure and elevated temperature in the presence of a catalyst which, in the non-reduced state, per 100 parts by weight of Cuo, comprises 40 to 130 parts by weight of ZnO, 2 to 50 parts by weight of Al₂O₃ and optionally 0.5 to 8 parts by weight of oxide of Mn, Mo, V, Zr and/or alkaline earth metal, has a BET total area of 80 to 175 m² per gram of catalyst in the non-reduced state, 75 to 95% of the BET total area being formed by pores having a radius rₚ≤ 15 nm.

2. The process as claimed in claim 1, wherein the active copper metal surface area of the reduced catalyst is 30 to 125, in particular 35 to 100, preferably 40 to 85 m² per gram of Cu.

3. The process as claimed in claim 1 or 2, wherein the catalyst in the non-reduced state, per 100 parts by weight of CuO, comprises 45 to 100, in particular 45 to 80 parts by weight of ZnO and 3 to 40, in particular 4 to 30, preferably 4 to 11 parts by weight of Al₂O₃.

4. The process as claimed in one or more of claims 1 to 3, wherein the catalyst has a BET total area of 85 to 160, in particular 90 to 155 m² per gram of catalyst in the non-reduced state.

5. The process as claimed in one or more of claims 1 to 4, wherein 80 to 92, in particular 84 to 90% of the BET total area are formed by pores having a radius rₚ≤ 15 nm.

6. The process as claimed in one or more of claims 1 to 5, wherein esters or fatty acids are reacted at from 200 to 350, in particular at from 220 to 330, preferably at from 230 to 320°C, and at from 15 to 40, in particular at from 18 to 35, preferably at from 20 to 32 MPa.

7. The process as claimed in one or more of claims 1 to 6, wherein esters of carboxylic acids, in particular of naturally occurring carboxylic acids, having from 8 to 30, in particular from 10 to 24, preferably from 12 to 22 carbon atoms, are reacted.

8. The process as claimed in one or more of claims 1 to 6, wherein fatty acids having from 8 to 30, in particular from 10 to 24, preferably from 12 to 22 carbon atoms, are reacted.

9. The process as claimed in one or more of claims 1 to 5, wherein nitriles are reacted at from 160 to 250, in particular at from 190 to 240, preferably at from 210 to 230°C, and at from 0.5 to 10, in particular at from 1.0 to 5.0, preferably at from 1.2 to 3.0 MPa.

10. The process as claimed in one or more of claims 1 to 5 and 9, wherein nitriles having from 4 to 30, in particular from 8 to 24, preferably from 10 to 22 carbon atoms, are reacted.

## Revendications

1. Procédé de préparation d'alcools ou d'amines par réaction d'esters, d'acides gras ou de nitriles avec l'hydrogène sous pression et à température élevée en présence d'un catalyseur qui, à l'état non réduit consiste en 100 parties en poids de CuO, 40 à 130 parties en poids de ZnO, 2 à 50 parties en poids d'Al₂O₃ et le cas échéant 0,5 à 8 parties en poids d'oxyde de Mn, de Mo, V, de Zr et/ou de métaux alcalino-terreux et a une surface totale BET de 80 à 175 m²/g de catalyseur à l'état non réduit, 75 à 95 % de la surface totale BET étant constitués de pores de rayon rₚ ≤ 15 nm.

2. Procédé selon la revendication 1, **caractérisé en ce que** la surface active du cuivre métallique du catalyseur réduit est de 30 à 125, plus spécialement de 35 à 100 et de préférence de 40 à 85 m²/g de Cu.

3. Procédé selon la revendication 1 ou 2, **caractérisé en ce que** le catalyseur à l'état non réduit consiste en 100 parties en poids de CuO, 45 à 100, plus spécialement 45 à 80 parties en poids de ZnO et 3 à 40, plus spécialement 4 à 30, de préférence 4 à 11 parties en poids d'Al₂O₃.

4. Procédé selon une ou plusieurs des revendications 1 à 3, **caractérisé en ce que** le catalyseur a une surface totale BET de 85 à 160, plus spécialement de 90 à 155 m²/g de catalyseur à l'état non réduit.

5. Procédé selon une ou plusieurs des revendications 1 à 4, **caractérisé en ce que** 80 à 92, plus spécialement 84 à 90 % de la surface totale BET sont constitués de pores d'un rayon rₚ ≤ 15 nm.

6. Procédé selon une ou plusieurs des revendications 1 à 5, **caractérisé en ce que** les esters ou acides gras sont convertis à 200 à 350, plus spécialement 220 à 330 et de préférence 230 à 320°C et à 15 à 40, plus spécialement 18 à 35 et de préférence 20 à 32 MPa.

7. Procédé selon une ou plusieurs des revendications 1 à 6, **caractérisé en ce que** l'on convertit des esters d'acides carboxyliques, plus spécialement d'acides carboxyliques natifs ayant 8 à 30, plus spécialement 10 à 24 et de préférence 12 à 22 atomes de carbone.

8. Procédé selon une ou plusieurs des revendications 1 à 6, **caractérisé en ce que** l'on convertit des acides gras ayant 8 à 30, plus spécialement 10 à 24 et de préférence 12 à 22 atomes de carbone.

9. Procédé selon une ou plusieurs des revendications 1 à 5, **caractérisé en ce que** l'on convertit les nitriles à 160 à 250, plus spécialement 190 à 240 et de préférence 210 à 230°C et à 0,5 à 10, plus spécialement 1,0 à 5,0 et de préférence 1,2 à 3,0 MPa.

10. Procédé selon une ou plusieurs des revendications 1 à 5 et 9, **caractérisé en ce que** l'on convertit des nitriles ayant 4 à 30, plus spécialement 8 à 24 et de préférence 10 à 22 atomes de carbone.
